# EUROPEAN PATENT APPLICATION

(11) **EP 4 364 864 A1**
(43) Date of publication of application: **08.05.2024**
(21) Application number: 23207977.2
(22) Date of filing: 06.11.2023
(51) Int. Cl.: B08B 9/032

(54) **SYSTEM AND METHOD FOR CLEANING MEAT PROCESSING EQUIPMENT AND MACHINERY**

(30) Priority: 07.11.2022 US 202218053320
(71) Applicant: Hantover, Inc., Overland Park, Kansas 66210 (US)
(72) Inventor: Curnett, Ronald J., Cross Timbers, Missouri, 65634 (US); Feeler, Craig E., Independence, Missouri, 64057 (US); Wheeler, Chad, Cedar Rapids, Iowa, 52402 (US)
(74) Representative: Uexküll & Stolberg

(57) **Abstract**

A system (10) and method for cleaning food processing equipment, which can include a plurality of individual containers (12), a plurality of conduits (14), fluid control components (18, 30), and a manifold (20). The individual containers contain different cleaning solutions (e.g., caustic, acid, and/or sanitizer). The conduits are each fluidly connected to either one of the plurality of individual containers or a water source (16), and the fluid control components are each configured to individually and selectively control when fluid flows through each of the conduits from one of the plurality of individual containers and/or the water source. The manifold has a plurality of inlets (22) each fluidly coupled with one of the plurality of conduits and one outlet (24) fluidly coupled with the plurality of inlets. The cleaning solutions mix with each other and/or with water within the manifold before being dispensed therefrom into food processing equipment.

## Description

### FIELD

The present invention generally relates to systems and methods for cleaning the interior and/or exterior surfaces of one or more components in a system.

### BACKGROUND

When animals are processed for human consumption, the various components of the animal must be cleaned. In addition to skeletal meat, other portions of the carcass that can be consumed directly by humans or used in the production of other foods is referred to as "offal." Animals frequently processed for human consumption can include beef, bovine, cattle, steers, heifers, cows, bulls, buffalo, pork, porcine, sows, gilts, barrows, boars, pigs, ovine, sheep, and lamb, for example.

Modern meat-processing plants use various food processing equipment and machinery to clean the offal prior to human consumption. The food processing equipment and machinery can include a cleaner (e.g., a cleaner for cleaning snout or tripe), a refiner such as a tripe refiner or a tongue refiner, a paunch washer, a washing machine for red offal, a deboning stand, a head splitting machine, an intestine cleaner, a hoof remover, a dehairing machine, or the like. Some of this equipment can be specially designed for specific animals or specific parts of the animals being processed.

In one example automated line for tripe cleaning, a loading lift with two buckets for joint input of paunches or bibles loads these animal components into a tripe washer for washing, scalding, and cleaning the inside of the paunches and bibles. Next to the tripe washer is a table for inspection and occasional handling of the cleaned animal components. From the table, these cleaned components are placed into a pneumatic lift for the loading of a tripe refiner located thereafter in the automated line. The tripe refiner is then used for defatting, refining, and finishing the tripe outer surfaces. An automatic panel can control this entire system, instructing various actuation and turning various components on and off. For example, the automatic panel can be programmed or otherwise configured to control lifting, washing, refining, thermostatic water mixtures, and the like.

To comply with sanitation standards for meat processing, this food processing equipment or offal-cleaning equipment and machinery must itself be thoroughly cleaned both inside and out at least daily, with the outside cleaned after each operation. Such cleaning also assists in making sure that the food processing equipment, such as offal-cleaning equipment, lasts a long time. However, cleaning of the offal-cleaning equipment traditionally requires additional laborers to perform this cleaning. Furthermore, some of the chemicals used in the cleaning process can be damaging to the offal-cleaning equipment.

For example, traditional cleaning methods for a refiner can include a worker closing a drain and/or run-off valves within a vat thereof, the worker adding disinfectant and detergent within the vat, the worker filling the vat with water, the worker running the refiner for a period of time, then the worker opening the drain or valve and rinsing using large quantities of water. The worker also may be required to brush the equipment. In addition to this process being time-consuming and labor-intensive, cleaning of such food processing equipment and machinery can be prone to human error if the procedures are not each carried out in a careful and consistent manner.

Accordingly, there is a need for systems and methods for cleaning the food processing equipment and machinery, such as offal-cleaning equipment, that overcomes the disadvantages of the prior art.

### SUMMARY

The present invention solves the above-described problems and provides a distinct advance in the art of cleaning food processing equipment and machinery, such as meat processing or offal processing equipment.

Specifically, one embodiment of the invention can include a system for cleaning food processing equipment, and can include a plurality of individual containers, a plurality of conduits, fluid control components, and a manifold. The plurality of individual containers can each contain different cleaning solutions (e.g., caustic, acid, and/or sanitizer). The plurality of conduits can each be fluidly connected to one of the plurality of individual containers or a water source, and the fluid control components may each be configured to individually and selectively control when fluid flows through each of the conduits from one of the plurality of individual containers and/or the water source. In some embodiments, the manifold has a plurality of inlets each fluidly coupled with one of the plurality of conduits and one outlet fluidly coupled with the plurality of inlets. The cleaning solutions mix with each other and/or with water within the manifold before being dispensed therefrom into food processing equipment.

In another embodiment, a method for cleaning food processing equipment can include a step of selectively dispensing water through a manifold into the food processing equipment. Furthermore, the method can include a step of selectively pumping or dispensing each of acid, caustic, and sanitizer into the manifold at different time intervals (e.g., water and caustic dispensed, later followed by water and acid being dispensed). The method can also include the steps of intermingling at least one of the acid, caustic, and sanitizer with the water within the manifold and outputting the intermingled water and the acid, caustic, and/or sanitizer out of the manifold into the food processing equipment.

This summary is intended to introduce a selection of concepts in a simplified form that are further described in the detailed description below. This summary is not intended to identify key features or essential features of the claimed subject matter, nor is it intended to be used to limit the scope of the claimed subject matter. Other aspects and advantages of the present invention will be apparent from the following detailed description of the embodiments and the accompanying drawing figures.

### BRIEF DESCRIPTION OF THE DRAWING FIGURES

Embodiments of the present invention are described in more detail below with reference to the attached drawing figures, wherein:
FIG. 1 is a schematic front view of a system for cleaning food processing equipment, in accordance with embodiments of the present invention;
FIG. 2 is a schematic front view of plumbing of the system of FIG. 1, in accordance with embodiments of the present invention;
FIG. 3 is a schematic air diagram of a multi-valve manifold of the system of FIG. 1, in accordance with embodiments of the present invention;
FIG. 4 is a schematic diagram of user input components and switches associated therewith for directing power to different components of a central controller of the system of FIG. 1, in accordance with embodiments of the present invention;
FIG. 5 is a schematic diagram of the central controller of FIG. 4 and other control circuitry of the system of FIG. 1, in accordance with embodiments of the present invention;
FIG. 6 is a schematic diagram of an exemplary refiner lid controllable by the system of FIG. 1, in accordance with embodiments of the present invention;
FIG. 7 is a flow chart of a method for cleaning food processing equipment, in accordance with embodiments of the present invention;
FIG. 8 is a timing diagram depicting a timing and sequence of a first exemplary recipe for operating the system of FIG. 1 in accordance with embodiments of the present invention;
FIG. 9 is a timing diagram depicting a timing and sequence of a second exemplary recipe for operating the system of FIG. 1 in accordance with embodiments of the present invention; and
FIG. 10 is a timing diagram depicting a timing and sequence of a third exemplary recipe for operating the system of FIG. 1 in accordance with embodiments of the present invention.

The drawing figures do not limit the present invention to the specific embodiments disclosed and described herein. The drawings are not necessarily to scale, emphasis instead being placed upon clearly illustrating the principles of the invention.

### DETAILED DESCRIPTION

The following detailed description of embodiments of the invention references the accompanying drawings. The embodiments are intended to describe aspects of the invention in sufficient detail to enable those skilled in the art to practice the invention. Other embodiments can be utilized and changes can be made without departing from the scope of the claims. The following detailed description is, therefore, not to be taken in a limiting sense. The scope of the present invention is defined only by the appended claims, along with the full scope of equivalents to which such claims are entitled.

In this description, references to "one embodiment", "an embodiment", or "embodiments" mean that the feature or features being referred to are included in at least one embodiment of the technology. Separate references to "one embodiment", "an embodiment", or "embodiments" in this description do not necessarily refer to the same embodiment and are also not mutually exclusive unless so stated and/or except as will be readily apparent to those skilled in the art from the description. For example, a feature, structure, act, etc. described in one embodiment may also be included in other embodiments but is not necessarily included. Thus, the present technology can include a variety of combinations and/or integrations of the embodiments described herein.

Systems and methods are described herein for cleaning food processing equipment such as meat processing equipment or offal processing equipment in an automated and/or semi-automated manner. The food processing equipment referenced herein can be any sort of food processing known in the art, such as meat processing equipment or offal processing equipment sold by HANTOVER INC. of Overland Park, Kansas. However, products made by other retails may be cleaned using the system and method herein without departing from the scope of the invention. The food processing equipment and machinery can include a cleaner (e.g., a cleaner for cleaning snout or tripe), a refiner such as a tripe refiner or a tongue refiner, a paunch washer, a washing machine for red offal, a deboning stand, a head splitting machine, an intestine cleaner, a hoof remover, a dehairing machine, or the like. Such food processing equipment or machinery can, in some cases, include a control system such as an automatic panel for control thereof, instructing various actuation and turning various components on and off. For example, the automatic panel can be programmed or otherwise configured to control lifting, washing, refining, thermostatic water mixtures, and the like.

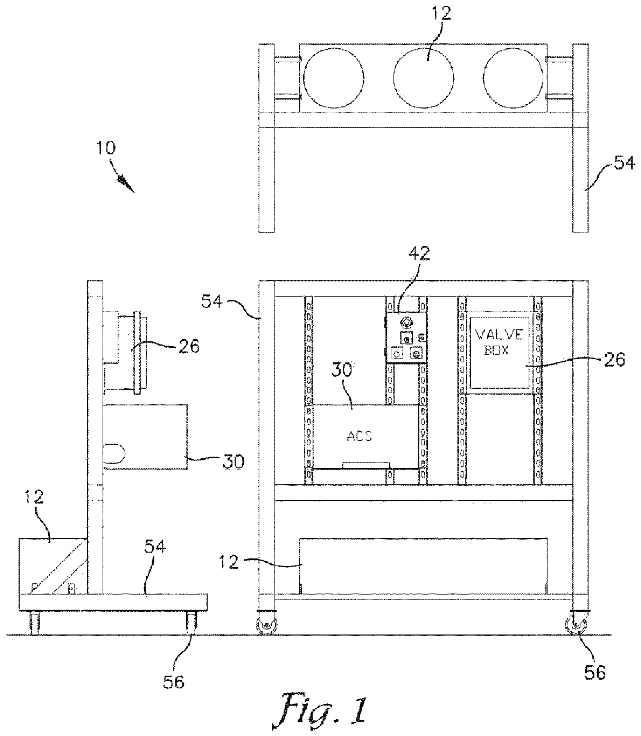

To clean such food processing equipment and machinery, a system 10 as depicted in FIGS. 1-5, includes a plurality of individual containers 12 each containing different cleaning solutions, a plurality of conduits 14 each fluidly connected to at least one of the individual containers 12 and/or a water source 16, fluid control components 18 each configured to individually and selectively control when fluid flows through each of the conduits 14 (e.g., from the individual containers 12 or the water source 16), and a manifold 20 having a plurality of inlets 22 each fluidly coupled with one of the conduits 14 and having one outlet 24 fluidly coupled with the inlets 22. The system can further comprise a central controller 26 configured for controlling timing of turning on and off each of the fluid control components 18 individually.

In some embodiments, the plurality of individual containers 12 can include three individual containers 12 each containing different cleaning solutions. The containers 12 can have any shape, size, or configuration suitable for holding their corresponding cleaning solutions therein in suitable quantities for cleaning of one or more pieces of food processing equipment. In one example embodiment, one of the individual containers 12 contains acid, one of the individual containers 12 contains caustic, and one of the individual containers 12 contains sanitizer. The acid or caustic can be foaming or non-foaming. The acid can be 1% of a total quantity of cleaning solution output into the food processing equipment. In some embodiments, the acid can include a sulfamic acid-based cleaner (e.g., KC-430 by PACKERS CHEMICAL of Kieler, Wisconsin), citric acid and hydroxyacetic acid-based solutions (e.g., KC-403 and/or KC-413 by PACKERS CHEMICAL of Kieler, Wisconsin), or the like. The caustic can be 2% of a total quantity of cleaning solution output into the food processing equipment and can include high alkaline cleaners containing highly potent bases such as potassium hydroxide and sodium hydroxide (e.g., KC-555 by PACKERS CHEMICAL of Kieler, Wisconsin), a high foaming caustic cleaner (e.g., KC-568 by PACKERS CHEMICAL of Kieler, Wisconsin), or the like. The sanitizer can be 500-800 parts part million (PPM) of the total quantity of cleaning solution output into the food processing equipment and can include antimicrobial solutions (e.g., KC-610 by PACKERS CHEMICAL of Kieler, Wisconsin), or the like. Other quantities and/or percentages of each of the different cleaning solutions described herein can be used without departing from the scope of the technology described herein. Furthermore, with foaming versions of these cleaning solutions, the system may be able to utilize less water and/or chemicals to achieve a desired level of cleanliness for the food processing equipment.

In some embodiments, the water source 16 can be part of the system 10 and/or fluidly coupled with the manifold 20 of the system 10. Water of different temperatures can be used during different stages of the methods described herein and/or these temperatures can be dependent upon the parts being cleaned and/or the purpose of the food processing equipment being cleaned. In some example embodiments, the water source 16 can include a source of hot water and a source of cold water with a lower temperature than the source of hot water. The central controller 26 can be programmed and configured to turn these separate sources of water on and off in accordance with one of the recipes stored therein, as later described herein. The water sources can each be coupled to separate inlets 22 of the manifold 20 and/or can include other mechanisms known in the art for switching between water of different temperatures. Furthermore, in some embodiments, timing/quantity of various chemicals / cleaning solutions output may vary based on local water supply alkalinity. Thus, some embodiments of the invention may additionally include sensors that incorporate such information into the recipes controlled by the central controller 26.

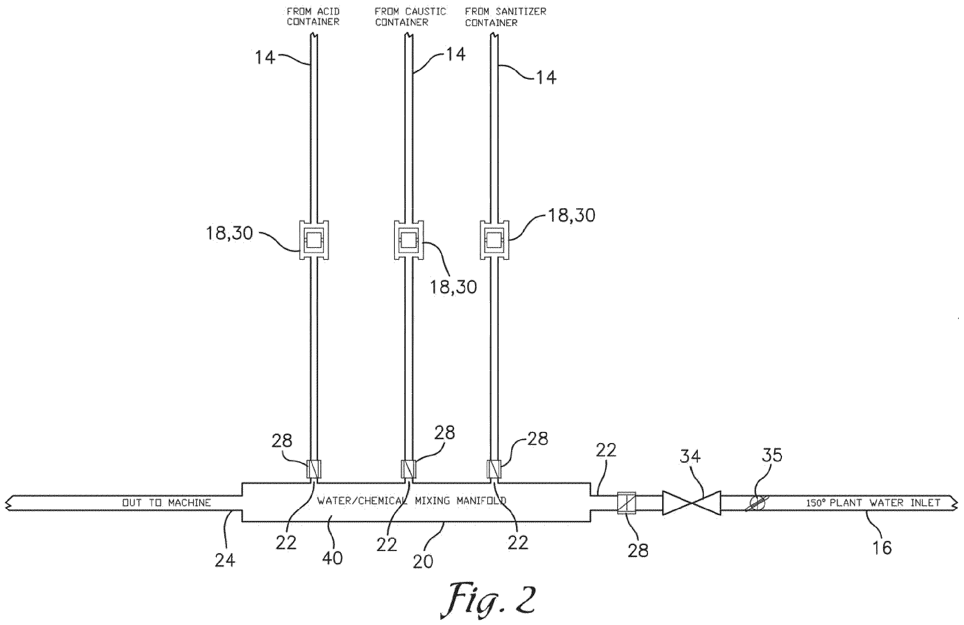

In some embodiments, as depicted in FIG. 2, the plurality of conduits 14 includes three conduits, each fluidly connecting the three individual containers 12 to the manifold 20. The conduits 14 can also include a fourth conduit connecting the water source 16 with the manifold 20, as well as a fifth conduit fluidly coupling the manifold 20 with the food processing equipment to be cleaned. Likewise, the plurality of fluid control components 18 can include three fluid control components 18 each configured to individually and selectively control when fluid (e.g., the cleaning solutions) flows through each of the conduits 14 associated therewith as well as a fourth fluid control component 18 for individually and selectively controlling when water is dispensed through one of the conduits 14. For example, the plurality of fluid control components 18 can comprise valves 28 actuatable between open and closed configurations to release the water or the cleaning solution from the individual containers, and/or pumps 30 configured to selectively pump the water and/or one of the different cleaning solutions out of one of the individual containers 12, through one of the conduits 14, and into the manifold 20.

In some embodiments, as depicted in FIG. 2, the pumps 30 are used to extract one of the cleaning solutions from its individual container 12, and a check valve (e.g., the valves 28) between the manifold 20 and the pumps 30 can assist in maintaining flow in a desired direction away from the individual container 12 and toward and into the manifold 20. Likewise, a check valve (e.g., the valves 28) can be located along the conduit 14 directing water into the manifold 20, as depicted in FIG. 2. The fluid control components 18 can be pneumatically controlled and/or can include, for example, solenoid valves (e.g., 120V 6.8W 57mA solenoid valve) or the like. Other fluid control components for selectively allowing and disallowing the cleaning solutions in the individual containers 12 to selectively flow into and selectively not flow into the manifold 20 can be used without departing from the scope of the technology described herein. In some embodiments, the system 10 can be configured for additional fluid control components 18 and/or actuator control components to provide for additional cleaning solutions or to actuate other components of the food processing equipment beyond the exemplary actuatable components described below.

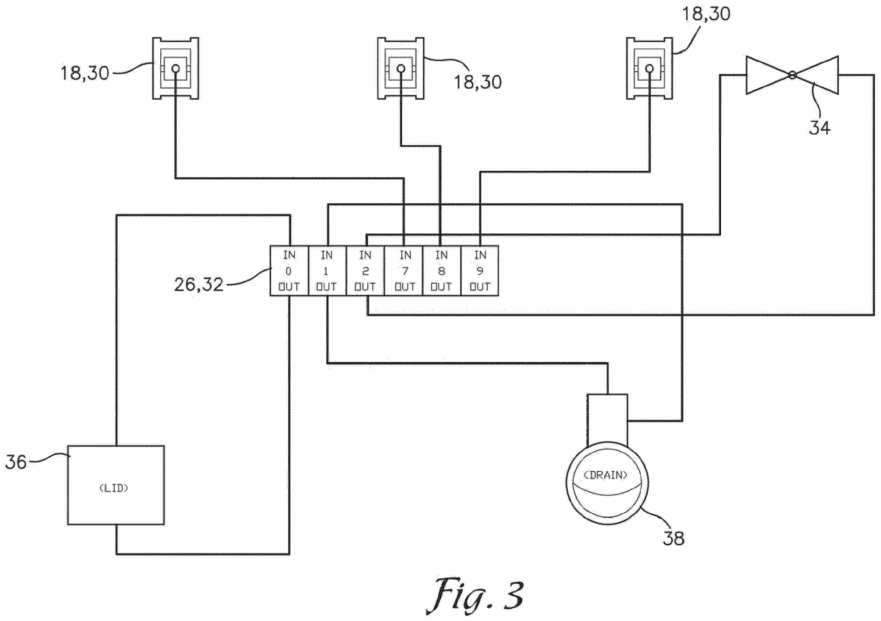

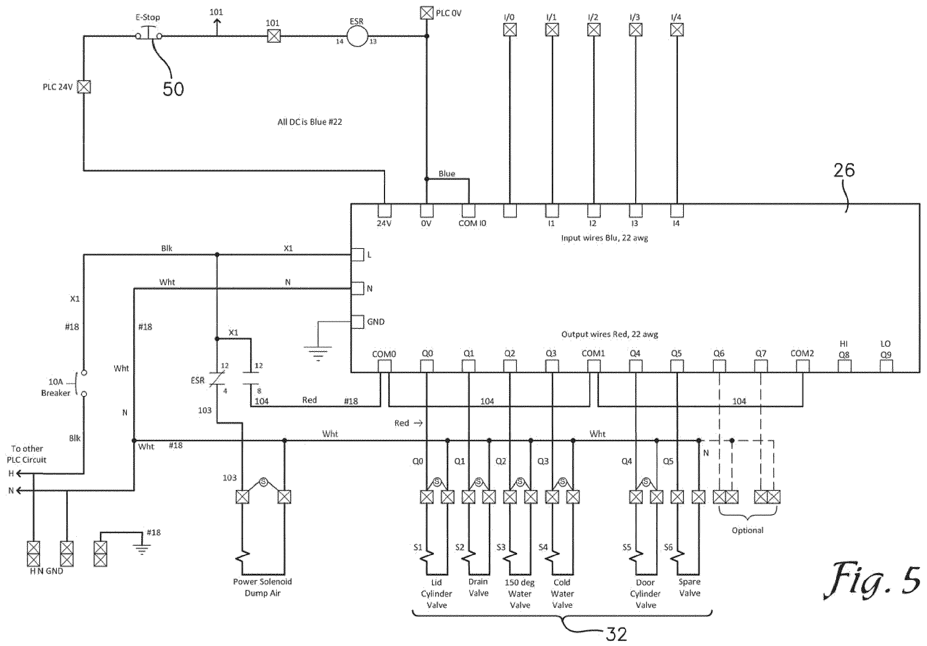

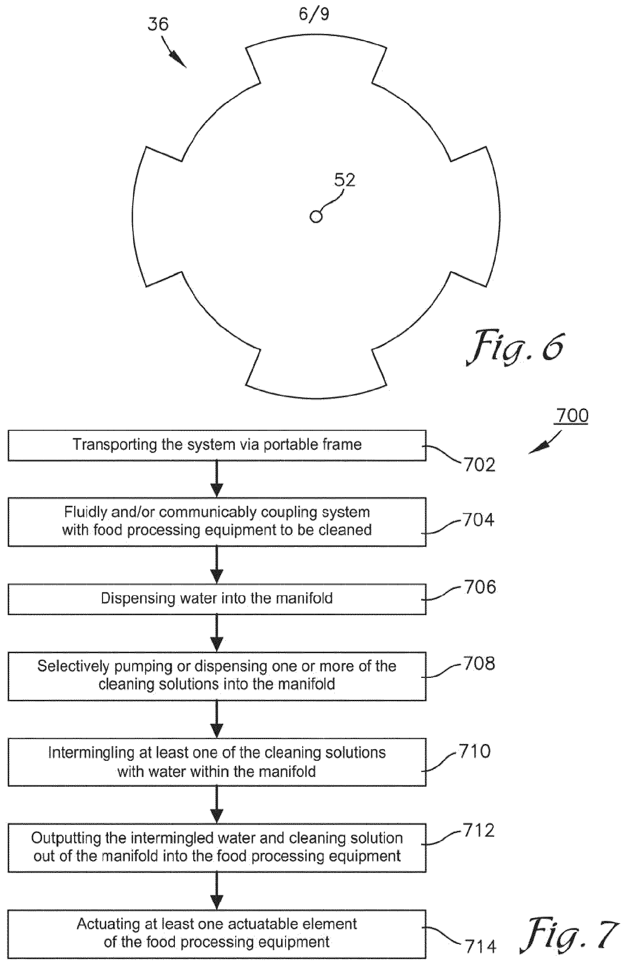

In one example embodiment, as depicted in FIG. 3, electronic air actuated valves 32 can be used by the central controller 26 to control the pumps 30, and other actuatable components of the system 10 and/or the food processing equipment, as described below. That is, the electronic air actuated valves 32 can be communicably and/or electrically coupled with the central controller 26 downstream from an acid pump, a caustic pump, and a sanitizer pump that are fluidly coupled with the conduits and the individual containers. Additionally, the water source 16 can be in fluid communication with an air actuated ball valve 34 and/or a manual ball valve 35 for allowing and disallowing water at predetermined intervals. Furthermore, in some embodiments, in addition to fluid control components 18, the system 10 can comprise actuator control components (e.g., electronic air actuated valves 32 associated with a lid and/or a drain) as also schematically depicted in FIG. 5. For example, in some embodiments, a lid 36 (depicted in FIG. 3 and 6) and/or a drain 38 (depicted in FIG. 3) can be actuated via the electronic air actuated valves 32 (as depicted in FIGS. 3 and 5).

The manifold 20, as depicted in FIG. 2, is configured for separately receiving each of the cleaning solutions and/or water, allowing any combination of these fluids to mix and/or comingle within the manifold 20, and outputting a resulting mixed or comingled fluid into and/or onto the food processing equipment for cleaning thereof. In some example embodiments, the plurality of inlets 22 comprises at least three inlets each fluidly coupled with one of the conduits 14 and may additionally comprise at least one other inlet 22 fluidly coupled with the water source 16. The one outlet 24 is fluidly coupled with the plurality of inlets 22, receiving the various liquids therefrom. Furthermore, an intermediate space 40 can be located between the inlets 22 and the outlet 24. The intermediate space 40 can be a chamber of any volume and/or shape. In some embodiments, the cleaning solutions and/or water are mixed with each other within the one outlet 24 and/or the intermediate space 40 between the inlets 22 and the outlet 24 before being dispensed therefrom into food processing equipment.

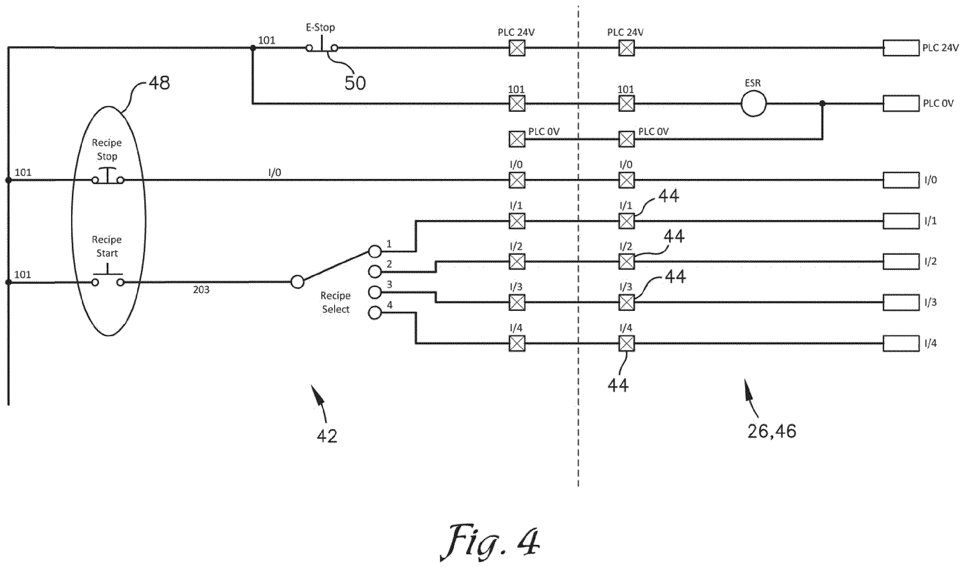

The central controller 26, as depicted in FIGS. 1 and 5, can include a processor, circuitry, memory, communication components, user input components 42, one or more displays, sensors, and/or a power supply or outlet connector. The user input components 42 may comprise, for example, a keyboard, a touchpad, a mouse, a mousepad, and/or a communications port for receiving user input from a wired or wireless remote user device. Additionally or alternatively, the user input components 42 can comprise switches (as depicted in FIG. 4), buttons, or other such user controls for controlling one or more of the following: power on, power off, emergency stop, recipe selection, recipe start, and/or recipe stop. The one or more displays can include, for example, an electronic screen or a physical dial associated with one or more readings/measurements. However, other user input components 42 and displays can be used as part of the central controller 26 without departing from the scope of the technology described herein.

The processor, circuitry, and/or memory of the central controller 26 may include any number of processors, controllers, integrated circuits, programmable logic controllers (PLCs), or other computing devices and resident or external memory for storing data and other information accessed and/or generated by various components of the system 10. The central controller 26 can include wired or wireless connections or communication components to enable information to be exchanged between the various components of the system 10 and/or the food processing equipment systems being cleaned by the system 10. In some embodiments the central controller 26 can be programmed with the sequence, duration, and/or other parameters for various actuatable components described herein for cleaning the food processing equipment. Such parameters to be programmed, controlled, and/or adjusted may include an amount of cleaning solution (e.g., acid, caustic, and sanitizer) to be pumped or dispensed, an amount of water to be dispensed, duration of dispensing the cleaning solutions and/or the water or start and stop times for dispensing the cleaning solutions and/or the water. However, other parameters can be programmed into the central controller 26 without departing from the scope of the technology described herein.

The central controller 26 and/or processors and memory thereof can implement a computer program and/or code segments to perform the functions described herein. The computer program may comprise an ordered listing of executable instructions for implementing logical functions in the central controller such as some of the steps illustrated in FIG. 7 and described below. For example, the computer program may be a software program configured to run on a computer, such as a personal computer, laptop, tablet, or the like. The computer program can be embodied in any computer-readable medium for use by or in connection with an instruction execution system, apparatus, or device, and execute the instructions. In the context of this application, a "computer-readable medium" can be any physical means that can contain, store, communicate, propagate, or transport the program for use by or in connection with the instruction execution system, apparatus, or device. The computer-readable medium can be, for example, but not limited to, an electronic, magnetic, optical, electro-magnetic, infrared, or semi-conductor system, apparatus, or device. More specific, although not inclusive, examples of the computer-readable medium would include the following: an electrical connection having one or more wires, a portable computer diskette, a random access memory (RAM), a read-only memory (ROM), an erasable, programmable, read-only memory (EPROM or Flash memory), a portable compact disk read-only memory (CDROM), an optical fiber, multi-media card (MMC), reduced-size multi-media card (RS MMC), secure digital (SD) cards such as microSD or miniSD, and a subscriber identity module (SIM) card. However, other types of processors and/or memory storage accessible by the processors described herein can be used without departing from the scope of the technology described herein.

In some example embodiments, as depicted in FIG. 4, the central controller 26 comprises a plurality of individual recipe PLCs 44 on a PLC panel 46. For example, the user input component 42 can be a switch that selectively directs power to the recipe PLCs 44 in the PLC panel 46, with each recipe PLC 44 programmed to output a different recipe or series of commands at different timing intervals to various actuatable components described herein. Likewise, a start/stop switch 48 and/or an emergency stop switch 50 can be coupled with a power source (e.g., a 24V power source or battery) to start or stop current from flowing into any of the recipe PLCs 44.

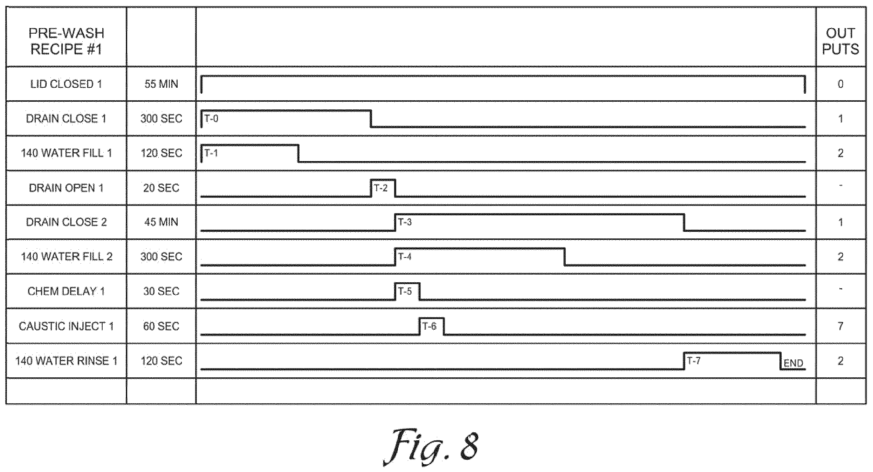

In some embodiments, the central controller 26 is configured to selectively control the timing of turning on and off each of the fluid control components in accordance with one or more of a plurality of user-selectable recipes, as depicted in FIGS. 8-10. In addition to turning on and off the fluid control components, the central controller 26 can, in some embodiments, be communicably coupled with at least one actuator (e.g., electronic air actuated valves 32 associated with a lid and/or a drain) for actuating at least one actuatable element of the food processing equipment (e.g., the lid 36 or drain 38). Control of the actuator control components can be performed, in some embodiments, via communication between the central controller 26 and a control system of the food processing equipment. Alternatively, the central controller 26 may be in direct communication with the actuators of the food processing equipment. For example, the at least one actuator on the food processing equipment can include: a drain actuator for selectively opening and closing the drain 38 of the food processing equipment based on instructions received from the central controller 26 and/or a lid actuator for opening and closing a lid (e.g., the lid 36 of a refiner as depicted in FIG. 6) of the food processing equipment based on instructions received from the central controller.

In some embodiments, the lid 36, as depicted in FIG. 6, can include a hole or port 52 that can be fluidly coupled via a hose or other such conduit to the outlet 24 of the manifold 20. The lid 36 can be slidably retractable and/or pivotally opened and closed. The lid actuator and/or the drain actuator can be pneumatic controllers or actuators. Parameters communicated to the control system of the food processing equipment from the central controller 26 of the system 10 described herein can include start times, stop times, duration, and the like. Note that while dispensing of water can be performed via conduits or hoses that are part of the system 10 and/or coupled with the manifold 20 described herein, in some alternative embodiments the water and/or additional water can be provided via conduits that are already part of the food processing equipment for its normal operations.

Note that some embodiments of the system 10 herein can be fixed in place or stationary (also referred to as "clean in place"), while an alternative embodiment can be configured for portable relocation throughout a meat processing plant. Such a portable system can be referred to as an assisted cleaning system (ACS). For example, some embodiments of the system 10 features a portable frame 54, as depicted in FIG. 1, with each of the individual containers 12, their conduits 14, the fluid control components 18, the manifold 20, the water source 16, and/or at least some components of the central controller 26 on the portable frame 54. The portable frame 54 can have any size, shape, and configuration. The portable frame 54 can be supported on wheels 56, rollers, a continuous track, and/or ball bearings. However, the portable frame 54 can additionally or alternatively utilize other mechanisms for allowing the system 10 to be transported from one location to another (e.g., throughout a plant or other such locations).

Advantageously, the cleaning solutions mix with water in the manifold 20, prior to contacting the food processing equipment, thereby protecting the equipment from even momentary contact with caustic or acid which may be damaging to some of the equipment. Likewise, various steps such as quantity, timing, sequences, and the like can be automated in accordance with one or more recipes that can be selected by a worker initiating the process. These recipes provide a consistent cleaning routine that precisely performs each step, in accordance with one or more of these recipes.

In use, a user can select one of the user-selectable recipes, the central controller 26 can instruct closing of the drain 38 and/or the lid 36 of the food processing equipment, and the central controller 26 can then pump and/or dispense the water and/or the cleaning solutions in accordance with the recipe selected. Then the drain 38 can be opened and/or closed by the central controller 26 at various pre-determined times or intervals during performance of the user-selected recipe. Likewise, each of the cleaning solutions and/or the water can be turned on and/or off at various times or intervals during the performance of the user-selected recipes via instructions form the central controller 26.

The flow chart of FIG. 7 depicts the steps of an exemplary method 700 for cleaning food processing equipment or machinery in more detail. In some embodiments of the invention, various steps may be omitted or steps may occur out of the order depicted in FIG. 7 without departing from the scope of the invention. For example, two blocks shown in succession in FIG. 7 may in fact be executed substantially concurrently, or blocks may sometimes be executed in the reverse order depending upon the functionality involved.

In some embodiments, the method 700 can include a step of transporting the system 10 via the portable frame 54, as depicted in block 702. This step of block 702 is specifically applicable for embodiments in which the system 10 is portable and can be omitted in alternative embodiments in which the system 10 is not portable. As described above, the portable frame 54 can be supported on wheels 56, rollers, a continuous track, or ball bearings, with the plurality of individual containers 12, the conduits 14, the fluid control components 18, at least a portion of the central controller 26, and the manifold 20 located on the portable frame 54. In some embodiments, the water source 16 can also be located on the portable frame 54. Transport of the portable frame 54 can be manual via a user pushing the cart and/or automated via remote control or other known methods of self-propulsion known in the art.

Furthermore, the method 700 can include a step of fluidly and/or communicably coupling portions of the system 10 with portions of the food processing equipment, as depicted in block 704. This method step can be performed whether or not the system 10 is portable. Specifically, the central controller 26 may be electrically and/or communicably coupled with components of the food processing equipment for operation/actuation thereof, and/or the outlet 24 of the manifold 20 can be fluidly coupled with the food processing equipment to be cleaned. For example, the port 52 in the lid 36 can be fluidly coupled with the outlet 24 of the manifold 20 or a hose leading from the outlet 24 to the port 52 in the lid 36 or into a top opening of a refiner vat, for example. Alternatively, the outlet 24 of the manifold 20 can be positioned directly above or in direct fluid connection with the food processing equipment to be cleaned.

The exemplary method 700 can further include the steps of dispensing water into the food processing equipment or machinery through the manifold 20, as depicted in block 706, and selectively pumping or dispensing one or more of the cleaning solutions into the manifold 20, as depicted in block 708. This includes pumping and/or otherwise dispensing the water and the cleaning solutions into the manifold 20 through the conduits 14 and inlets 22 described above. In some embodiments, the water and/or the cleaning solutions (e.g., acid, caustic, and sanitizer) are dispensed in accordance with user-selectable recipes and/or any recipe/sequence pre-programed into the central controller 26, such as those depicted in FIGS. 8-10. Thus, steps 706 and 708 can be accomplished in an automated manner by the central controller 26 in a variety of orders and combinations. For example, the pumping and/or dispensing step of block 708 can include the central controller 26 turning on and off each of the fluid control components 18 (e.g., pumps or valves) independently in accordance with one of at least two stored recipes for selectively pumping or dispensing the water, acid, caustic, and sanitizer into the manifold 20. Each of the at least two stored recipes can include a predetermined time and/or sequence controlling the pumps and/or valves described above.

Furthermore, the method 700 includes a step of intermingling at least one of the cleaning solutions (e.g., the acid, caustic, and sanitizer) with water within the manifold 20, as depicted in block 710. The intermingling step in block 710 can, in some embodiments, include intermingling the cleaning solutions with the water in the manifold 20 in the intermediate space 40 between the inlets 22 and the outlet 24 and/or within the outlet 24 itself. This intermingling within the manifold 20 prevents straight caustic or straight acid, for example, from being dispensed directly into the food processing equipment, thereby protecting the food processing equipment from rapid deterioration (often associated with conventional processes). For example, caustic on its own can cause calcium build up within a refiner that can be damaging to the refiner (e.g., the stone plate within the refiner), but by first dispensing the water and then mixing the caustic with the water within the manifold, the caustic is not dispensed until it is intermingled with the water and the refiner is less prone to damage during this cleaning process.

The method 700 also includes a step of outputting the intermingled water and cleaning solutions (e.g., acid, caustic, and/or sanitizer) out of the manifold into the food processing equipment, as depicted in block 712. The input of fluids into the manifold 20 and their resulting output therefrom is controlled via the fluid control components 18 described above. As noted above, in some embodiments, the manifold 20 is sized and configured to allow for sufficient intermingling or mixing of the cleaning solutions naturally therein prior to exiting through the outlet into the food processing equipment.

The method 700 can also include, in some embodiments, a step of the central controller 26 instructing at least one actuator to actuate at least one actuatable element (e.g., a lid or drain) of the food processing equipment, as depicted in block 714. For example, the central controller 26 can be communicably and/or electronically coupled with a control system for the food processing equipment, such as the control system for a refiner. The central controller 26 can provide instructions, for example, to open and close the drain 38 of the food processing equipment via a drain actuator for selectively opening and closing the drain 38. The sequence in which this drain 38 opens and closes, and the timing thereof, can be part of an overall recipe selected by the user. These instructions can be provided by way of the central controller 26 communicating with the control system of the food processing equipment and/or can be accomplished via direct communication between the central controller 26 and the drain actuator or the lid actuator.

For example, in some embodiments, the central controller 26 can provide instructions to open and close a lid (e.g., a lid of a refiner) via a lid actuator. The sequence in which the lid 36 is opened and closed, and the timing thereof can be part of an overall recipe selected by the user. These instructions can be provided by way of the central controller 26 communicating with the control system of the food processing equipment and/or can be accomplished via direct communication between the central controller 26 and the lid actuator. The lid 36 can be a lid for a washer, a refiner, dehairing equipment, or the like and may be used to prevent the materials placed into and processed therein from splashing outwards. In some embodiments, instructions to lock or unlock the lid 36 can additionally or alternatively be conveyed by the central controller 26 to locks (not shown) of the lid 36. Other such actuatable features of the food processing equipment not listed or depicted herein can also be controlled via the central controller 26 and the system 10 described herein in a manner identical or similar to the way the lid 36 and the drain 38 are actuated.

Several example assisted cleaning recipes are depicted in timing on/off charts in FIGS. 8-10. For example, the pre-wash recipe of FIG. 8 (also identified as "RECIPE #1") starts with the lid closed, the drain closed, and the water filling a refiner vat. The drain is then opened to conclude a "fill and flush" stage of this pre-wash recipe. Then the drain is closed again, the water fills the refiner vat, and after a delay labeled "CHEM DELAY 1," the caustic is output into the manifold and dispensed out therefrom along with the water. The water continues to fill the refiner vat for a time after the required amount of caustic is dispensed. In some embodiments, the refiner vat is run or operated with this mixture therein, which operation can be actuated by the central controller of the system 10 disclosed above and/or can be manually turned on by an operator via controllers of the refiner vat. This pre-wash can last a total of fifty five (55) minutes in this example Recipe #1, with ending with the drain opening and water rinsing out the refiner vat.

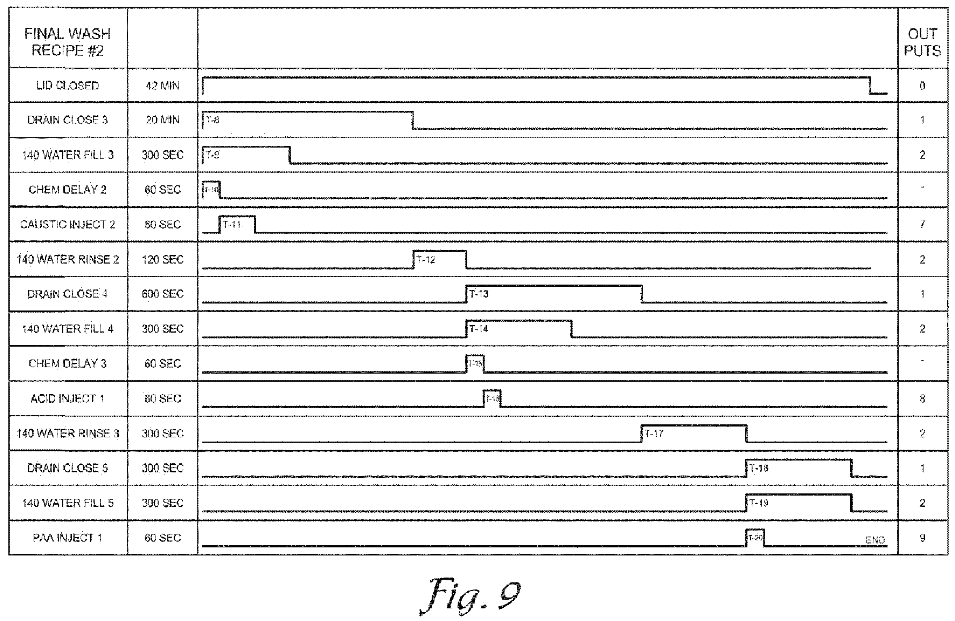

In an example Recipe #2, as depicted in FIG. 9, a final wash can serve as any one of the user selectable recipes and may include adding water and caustic simultaneously via the manifold (with water starting first and then caustic added after a short delay time). This can be referred to as a caustic cycle in which caustic is added to the manifold and/or the food processing equipment (e.g., the refiner vat). This caustic cycle can then be followed by a rinse cycle with the drain opened, and then when the drain is closed again, an acid cycle includes water being added simultaneously with acid (again with the water starting first and then acid being added after a short delay time). Next, following yet another rinse cycle, a sanitizer cycle can commence. Note that the sanitizer added in FIG. 9 is referred to as "PAA INJECT," however any sanitizer can be used without departing from the scope of the technology described herein. The sanitizer cycle in this example does not include a time delay, with the water and sanitizer being added simultaneously and the water continuing until the refiner vat is full (even after a desired quantity of sanitizer is added thereto). However, note that in other recipes there can still be a delay time between when the water begins to be dispensed and when the sanitizer is added without departing from the scope of the technology described herein.

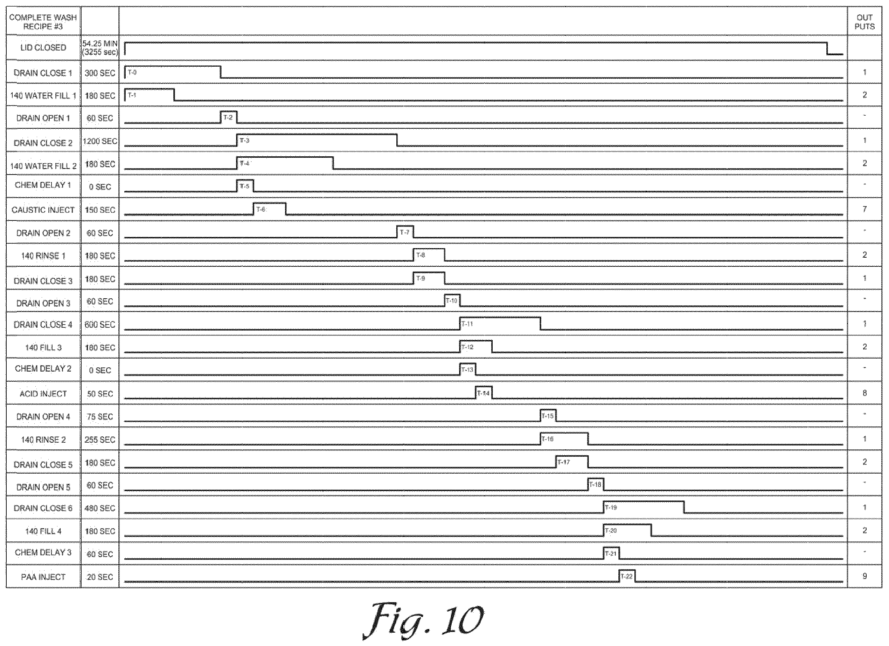

Additionally or alternatively, as depicted in FIG. 10, Recipe #3, a complete wash without foam can serve as any one of the user selectable recipes. For example, the complete wash without foam can include a flush stage followed by a caustic cycle which is then followed by a rinse cycle, similar to the pre-wash recipe above. Following the rinse cycle of this pre-wash portion of the complete wash, an acid cycle similar to the caustic cycle may commence, followed by yet another rinse cycle, and then a sanitizer cycle. Note that the sanitizer added in FIG. 10 is referred to as "PAA INJECT," however any sanitizer can be used without departing from the scope of the technology described herein.

Although the invention has been described with reference to example embodiments illustrated in the attached drawing figures, it is noted that equivalents may be employed and substitutions made herein without departing from the scope of the invention as described and claimed herein.

## Claims

1. A system for cleaning food processing equipment, the system comprising:
a plurality of individual containers each containing different cleaning solutions;
a plurality of conduits, wherein each of the plurality of individual containers is fluidly connected to one of the plurality of conduits and a water source is fluidly connected to at least one of the plurality of conduits;
fluid control components each configured to individually and selectively control when fluid flows through each of the conduits from one of the plurality of individual containers or the water source; and
a manifold having a plurality of inlets each fluidly coupled with one of the plurality of conduits and one outlet fluidly coupled with the plurality of inlets, wherein water and at least one of the cleaning solutions mix with each other within the manifold and flow into the food processing equipment.

2. The system of claim 1, wherein one of the plurality of individual containers contains acid, one of the plurality of individual containers contains caustic, and one of the plurality of individual containers contains sanitizer.

3. The system of claim 1, further comprising a central controller configured for controlling timing of turning on and off each of the fluid control components individually.

4. The system of claim 3, wherein the central controller is configured to selectively control the timing of turning on and off each of the fluid control components in accordance with one or more of a plurality of user-selectable recipes.

5. The system of claim 3, wherein the central controller is communicably coupled with at least one actuator for actuating at least one actuatable element of the food processing equipment.

6. The system of claim 5, wherein the at least one actuator includes at least one of:
a drain actuator for selectively opening and closing a drain of the food processing equipment based on instructions received from the central controller, and
a lid actuator for opening and closing a lid of a refiner of the food processing equipment based on instructions received from the central controller.

7. The system of claim 1, further comprising the water source, wherein the water source includes a source of hot water and a source of cold water with a lower temperature than the source of hot water.

8. The system of claim 1, wherein the fluid control components each comprise at least one of:
a valve actuatable between open and closed configurations, and
a pump configured to selectively pump one of the different cleaning solutions out of the plurality of individual containers.

9. The system of claim 1, further comprising a portable frame supported on wheels, rollers, a continuous track, or ball bearings, wherein the plurality of individual containers, the plurality of conduits, the fluid control components, and the manifold are located on the portable frame.

10. A method for cleaning food processing equipment, the method comprising:
selectively dispensing water through a manifold into the food processing equipment;
selectively pumping or dispensing each of acid, caustic, and sanitizer into the manifold at different time intervals;
intermingling at least one of the acid, caustic, and sanitizer with the water within the manifold; and
outputting the intermingled water and the at least one of acid, caustic, and sanitizer out of the manifold into the food processing equipment.

11. The method of claim 10, wherein the step of dispensing water occurs at a predetermined interval of time prior to selectively pumping or dispensing at least one of the acid, caustic, and sanitizer.

12. The method of claim 10, wherein the step of selectively pumping or dispensing is automated by a central controller.

13. The method of claim 12, further comprising the central controller turning on and off each of a plurality fluid control components independently in accordance with one of at least two stored recipes for selectively pumping or dispensing the acid, caustic, and sanitizer into the manifold, wherein each of the at least two stored recipes includes a predetermined time and sequence.

14. The method of claim 13, wherein the central controller selectively controls the dispensing of the water into the food processing equipment through the manifold in accordance with the one of the at least two stored recipes.

15. The method of claim 12, further comprising the central controller instructing at least one actuator to actuate at least one actuatable element of the food processing equipment.
